# EUROPEAN PATENT SPECIFICATION

(11) **EP 3 532 707 B1**
(45) Date of publication and mention of the grant of the patent: **04.12.2024**
(21) Application number: 17863345.9
(22) Date of filing: 31.10.2017
(51) Int. Cl.: E21B 49/08, G01N 1/20, G01N 33/28, G01N 1/14, G01N 1/10

(54) **METHODS AND SYSTEMS FOR SAMPLING AND/OR ANALYZING FLUID, SUCH AS PRODUCTION FLUID FROM AN OIL AND GAS WELL**
VERFAHREN UND SYSTEME ZUR PROBENAHME UND/ODER ANALYSE EINER FLÜSSIGKEIT WIE EINER PRODUKTIONSFLÜSSIGKEIT AUS EINEM ÖL- UND GASBOHRLOCH
PROCÉDÉS ET SYSTÈMES POUR ÉCHANTILLONNER ET/OU ANALYSER UN FLUIDE, TEL QU'UN FLUIDE DE PRODUCTION PROVENANT D'UN PUITS DE PÉTROLE ET DE GAZ

(30) Priority: 31.10.2016 US 201662415147 P
(43) Date of publication of application: 04.09.2019
(73) Proprietor: Abu Dhabi National Oil Company (ADNOC), 00971 Abu Dhabi (AE)
(72) Inventor: YAN, Chao, Sugarland, Texas 77479 (US); GHORBANI, Nasser, The Woodlands, Texas 77380 (US); GURAIEB, Paula, Houston, Texas 77098 (US); TOMSON, Ross, Houston, Texas 77096 (US)
(74) Representative: Mader, Joachim
(86) International application number: PCT/IB2017/056765
(87) International publication number: WO 2018/078609

(56) References cited:
- EP-A1- 2 179 135
- EP-A2- 2 549 057
- WO-A1-2016/118802
- US-A- 4 994 671
- US-A1- 2007 209 793
- US-A1- 2009 293 605
- US-A1- 2011 303 409
- US-A1- 2013 075 093
- US-A1- 2015 007 648
- US-A1- 2016 238 531

## Description

### BACKGROUND

### 1. Field of Invention

The present invention relates generally to methods and systems for sampling and/or analyzing fluid, which can be used to, for example, detect and/or predict scale formation on equipment, transmission lines, and/or the like that handle the fluid. Non-limiting examples of fluids suitable for sampling and/or analyzing with the present invention include production fluid from an oil and gas well, another hydrocarbon-containing fluid (*e.g.,* in an upstream, midstream, and/or downstream process), industrial process water (*e.g.,* boiler water, cooling tower water, waste water, or the like), or the like.

### 2. Description of Related Art

Many methods for detecting and/or predicting scale formation on equipment, transmission lines, and/or the like that handle fluid involve retrieving samples of that fluid and transporting those samples off-site for analysis. Due to depressurization of the samples, lowering of temperature of the samples, degassing, precipitation, the time between retrieving and analyzing the samples, attempts made to preserve the samples, and/or the like, the samples may no longer be representative of the fluid when they are analyzed, leading to erroneous scale formation predictions and ineffective scale management.
Document EP 2 549 057 A2 discloses a sampling tool to sample formation fluids in a wellbore. The sampling tool may include a sample chamber having a fluid inlet port and a tubular portion. A first piston may be sealably and movably disposed within the tubular portion. One or more surfaces of the first piston and the sample chamber may, at least in part, define a sample space. A second piston may be sealably and movably disposed within the first piston.

### SUMMARY

The invention is defined in independent claims. Dependent claims concern particular embodiments of the invention. Any subject matter presented in the description, for example, as an embodiment, but not falling under the claims constitutes an aspect of the disclosure which may be useful for understanding the invention.

Embodiments of the present invention can be used to obtain a sample of a fluid, and, in some instances, analyze that sample, without undesirably altering the sample's pressure; in at least this way, changes to the sample's properties (e.g., due to degassing, precipitation, and/or the like) can be mitigated that might otherwise render the sample unrepresentative of the fluid. In some embodiments, such desirable functionality can be achieved through use of: (1) a container coupled to a conduit through which the fluid is conveyed, the container having a first chamber in fluid communication with the conduit, a second chamber, and a movable divider disposed between and in fluid communication with each of the first and second chambers; and (2) a pressure source in fluid communication with the second chamber, where the pressure source can be controlled such that a difference in pressure between the first and second chambers is sufficient to draw a sample of the fluid into the first chamber but small enough to avoid undesirably changing the sample's pressure (exemplary values for such chamber pressures are provided below). Similarly, in some such embodiments, the sample can then be directed from the first chamber to one or more sensors for analysis by controlling the pressure source such that a difference in pressure between the first and second chambers is sufficient to expel the sample from the first chamber but small enough to avoid undesirably changing the sample's pressure.

Further, via such two-chamber, pressure-responsive operation, the container may respond more quickly to pressure changes within the conduit, be more capable of absorbing pressure fluctuations, and/or the like than other sampling containers, such as those including mechanically-driven pistons, and therefore may be better suited for obtaining a fluid sample without undesirably altering its pressure.

Some embodiments can mitigate changes in the temperature of the sample; for example, in such embodiments, the temperature of the sample within the container can be maintained at one that is substantially equal to the temperature of the sample prior to being received by the container. Such functionality can be provided by, for example, a heating element that heats the container and/or other structure(s) that contact the sample. As with maintaining its pressure, maintaining the temperature of the sample can avoid undesirable changes to the sample's properties.

Some embodiments of the present methods for sampling and/or analyzing fluid (e.g., production fluid from an oil and gas well) can include: receiving, into a container coupled to a fluid conduit *(e.g.,* production fluid conduit), fluid *(e.g.,* production fluid) from the fluid conduit, wherein the container comprises a housing defining an interior volume and a divider movably disposed within the housing and dividing the interior volume into a first chamber and a second chamber, and wherein the receiving comprises reducing pressure within the second chamber to draw production fluid into the first chamber. In some methods, the divider comprises a piston or a flexible bladder.

In some methods, prior to the receiving, pressure within the second chamber is substantially equal to a pressure of fluid (e.g., production fluid) within the fluid conduit fluid conduit (*e.g.,* production fluid conduit), and the reducing pressure within the second chamber comprises reducing pressure within the second chamber by between approximately 1 pound per square inch (psi) and approximately 10 psi. In some methods, the reducing pressure within the second chamber is performed using a pump and/or a regulator in fluid communication with the second chamber.

In some methods, the receiving fluid (*e.g.,* production fluid) has a first pressure that is substantially equal to a pressure of fluid (*e.g.,* production fluid) within the fluid conduit (*e.g.,* production fluid conduit), and the method comprises separating, within the container, the received fluid (*e.g.,* production fluid) into an oil and/or gas portion and a water portion having a higher water content than that of the oil and/or gas portion, and capturing, with one or more sensors, data indicative of one or more properties of the water portion, wherein the water portion, during the capturing, has a pressure that is substantially equal to the first pressure. In some methods, the received fluid (*e.g.,* production fluid) has a first temperature that is substantially equal to a temperature of fluid (*e.g.,* production fluid) within the fluid conduit (*e.g.,* production fluid conduit), and the water portion, during the capturing, has a temperature that is substantially equal to the first temperature. Some methods comprise heating the container using a heating element.

In some methods, the separating comprises retaining the received fluid (*e.g.,* production fluid) within the container for a period of time. In some methods, the period of time is between approximately 5 minutes and approximately 60 minutes. In some methods, the separating comprises providing a demulsifier to the received fluid (*e.g.,* production fluid).

Some methods comprise directing at least a first portion of the received fluid (*e.g.,* production fluid) to at least one of the conduit (*e.g.,* production fluid conduit) and a reservoir and at least a second portion of the received fluid (*e.g.,* production fluid) to the one or more sensors, wherein the directing is performed based, at least in part, on data captured by a sensor, the data being indicative of a water content of the first portion and/or the second portion. In some methods, the sensor comprises an optical sensor.

In some methods, the one or more properties comprise a total dissolved solids (TDS) content, a pH, an alkalinity, a total hardness, a hydrogen sulfide content, an ammonia content, a hydrocarbon content, a carbon dioxide content, a total metal carbonate content, a total metal sulfate content, a total metals content, a sodium chloride content, a silicate content, an iron content, a calcium content, a sodium content, a magnesium content, a potassium content, a strontium content, a chlorine content, a chloride content, a bicarbonate content, a phosphorous content, a boron content, a barium content, a sulfate content, an iron content, a nickel content, a chromium content, a cobalt content, a molybdenum content, a specific gravity, a conductivity, a saturation index and/or ratio, a resistivity, a pressure, and/or a temperature.

In some methods, the one or more sensors comprises a spectrophotometer. In some methods, the one or more sensors comprises a pH probe. In some methods, the one or more sensors comprises a conductivity probe. In some methods, the one or more sensors comprises an ion-selective electrode.

Some methods comprise calibrating at least one of the one or more sensors at least by capturing, with the at least one sensor, data indicative of one or more properties of a fluid, wherein at least one of the one or more properties of the fluid is known. In some methods, the fluid comprises a stock solution and/or diluent. In some methods, the stock solution and/or the diluent comprises water, an alcohol, a glycol, a mineral acid, an organic acid, a buffer, and/or ammonium hydroxide.

Some methods comprise providing one or more diluents to the water portion. Some methods comprise providing one or more reagents to the water portion. In some methods, at least one of the one or more reagents is responsive to pH and/or alkalinity and comprises thymol blue, methyl red, bromothymol blue, bromocresol green, bromocresol purple, and/or phenolphthalein. In some methods, at least one of the one or more reagents is responsive to iron and comprises 1,10-phenanthroline, 4,7-diphenyl-1,10-phenanthroline, 2,4,6-tris(2-pyridyl)-1,3,5triazine, 2,2 bypyridine, potassium cyanide, and/or 2,2',2" tripyridine. In some methods, at least one of the one or more reagents comprises a chelating agent. In some methods, the chelating agent comprises ethylenediaminetetraacetic acid (EDTA), nitrilotriacetic acid (NTA), disuccinic acid, glucoheptonate, monoethanolethylenediamine triacetic acid, diethylenatriamine pentacetic acid, and/or citric acid.

The invention comprises sampling and/or analyzing production fluid from an oil and gas well comprise: receiving, into a first chamber of a container coupled to a production fluid conduit, production fluid from the production fluid conduit, the container having a second chamber and a movable divider disposed between and in fluid communication with each of the first chamber and the second chamber, wherein receiving production fluid into the first chamber comprises drawing production fluid into the first chamber at least by controlling a pressure source in fluid communication with the second chamber such that a force acting on the divider due to pressure within the second chamber is reduced to, and remains as production fluid is drawn into the first chamber, less than but within 10% of a force acting on the divider due to pressure within the first chamber.

In some methods, prior to drawing production fluid into the first chamber, pressure within the second chamber is greater than and/or substantially equal to pressure within the first chamber. In the invention, controlling the pressure source to draw production fluid into the first chamber is performed such that, as production fluid is drawn into the first chamber, pressure within the second chamber remains less than but within 10% of pressure within the first chamber. In some methods, controlling the pressure source to draw production fluid into the first chamber is performed such that, as production fluid is drawn into the first chamber, a difference between pressure within the first chamber and pressure within the second chamber remains less than approximately 10 psi. In some methods, the pressure source comprises a pump and/or controlling the pressure source comprises controlling a regulator in fluid communication with the pressure source.

The invention comprises capturing, with one or more sensors, data indicative of one or more properties of at least a portion of the received production fluid. The invention comprises expelling at least a portion of the received production fluid from the first chamber at least by controlling the pressure source such that a force acting on the divider due to pressure within the second chamber is greater than a force acting on the divider due to pressure within the first chamber, and directing at least a first portion of the expelled production fluid to the one or more sensors. The invention comprises controlling the pressure source to expel the portion of the received production fluid is performed such that, as the portion of the received production fluid is expelled from the first chamber, a force acting on the divider due to pressure within the second chamber remains within 10% of a force acting on the divider due to pressure within the first chamber.

The invention comprises a system for sampling and analyzing production fluid from an oil and gas well comprise: a container having a housing defining an interior volume and a divider movably disposed within the housing and dividing the interior volume into a first chamber and a second chamber, the first chamber configured to be in fluid communication with a production fluid conduit, and a pressure source configured to be in fluid communication with and to vary a pressure within the second chamber, wherein, when the first chamber is in fluid communication with the production fluid conduit, when pressure within the second chamber is lower than that within the first chamber, production fluid is conveyed from the production fluid conduit and into the first chamber, and, when pressure within the second chamber is higher than that within the first chamber, production fluid is conveyed from the first chamber and into the production fluid conduit. In some systems, the divider comprises a piston or a flexible bladder. In some systems, the pressure source comprises a pump and/or a regulator. In some systems, the system is coupled to a wellhead.

The invention comprises a first conduit configured to be in fluid communication with the first chamber and to convey production fluid from the first chamber and to one or more sensors, wherein the one or more sensors are configured to capture data indicative of one or more properties of the production fluid. Some systems comprise a second conduit configured to be in fluid communication with the first chamber and to convey production fluid from the first chamber and to at least one of the production fluid conduit and a reservoir. Some systems comprise one or more valves configured to control fluid communication through the first and second conduits and a sensor configured to capture data indicative of a water content of production fluid within the second conduit, wherein the one or more valves are configured to be actuated to block fluid communication through the second conduit and allow fluid communication through the first conduit based, at least in part, on data captured by the sensor. In some systems, the sensor comprises an optical sensor.

In some systems, the one or more properties of the production fluid comprises a TDS content, a pH, an alkalinity, a total hardness, a hydrogen sulfide content, an ammonia content, a hydrocarbon content, a carbon dioxide content, a total metal carbonate content, a total metal sulfate content, a total metals content, a sodium chloride content, a silicate content, an iron content, a calcium content, a sodium content, a magnesium content, a potassium content, a strontium content, a chlorine content, a chloride content, a bicarbonate content, a phosphorous content, a boron content, a barium content, a sulfate content, an iron content, a nickel content, a chromium content, a cobalt content, a molybdenum content, a specific gravity, a conductivity, a saturation index and/or ratio, a resistivity, a pressure, and/or a temperature.

In some systems, the one or more sensors comprises a spectrophotometer. In some systems, the one or more sensors comprises a pH probe. In some systems, the one or more sensors comprises a conductivity probe. In some systems, the one or more sensors comprises an ion-selective electrode.

In some systems, the pressure source comprises a pump and/or the processor is configured to control the pressure source by controlling a regulator in fluid communication with the pressure source.

Some systems include one or more sensors configured to capture data indicative of a difference between a force acting on the divider due to pressure within the second chamber and a force acting on the divider due to pressure within the first chamber, wherein the processor is configured to control the pressure source based, at least in part, on data captured by the one or more sensors. In some systems, the one or more sensors include a sensor configured to capture data indicative of pressure within the second chamber and/or a sensor configured to capture data indicative of pressure within the first chamber.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; *e.g.,* substantially 90 degrees includes 90 degrees and substantially parallel includes parallel), as understood by a person of ordinary skill in the art. In any disclosed embodiment, the terms "substantially" and "approximately" may be substituted with "within [a percentage] of" what is specified, where the percentage includes .1, 1, 5, and 10 percent.

The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

Further, a device or system that is configured in a certain way is configured in at least that way, but it can also be configured in other ways than those specifically described.

The terms "comprise" (and any form of comprise, such as "comprises" and "comprising"), "have" (and any form of have, such as "has" and "having"), "include" (and any form of include, such as "includes" and "including"), and "contain" (and any form of contain, such as "contains" and "containing") are open-ended linking verbs. As a result, an apparatus that "comprises," "has," "includes," or "contains" one or more elements possesses those one or more elements, but is not limited to possessing only those one or more elements. Likewise, a method that "comprises," "has," or "includes," or "contains" one or more steps possesses those one or more steps, but is not limited to possessing only those one or more steps.

Any embodiment of any of the apparatuses, systems, and methods can consist of or consist essentially of - rather than comprise/have/include/contain - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

The feature or features of one embodiment may be applied to other embodiments, even though not described or illustrated, unless expressly prohibited by this disclosure or the nature of the embodiments.

Some details associated with the embodiments are described above, and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers.
**FIG. 1** is a schematic of a first embodiment of the present systems for sampling and analyzing production fluid from an oil and gas well.
**FIG. 2** is a schematic of a container for receiving a sample of production fluid from a production fluid conduit.
**FIG. 3** is a schematic of a sensor for detecting one or more properties of production fluid.
**FIG. 4** is a schematic of a second embodiment of the present systems for sampling and analyzing production fluid from an oil and gas well.
**FIG. 5** is a schematic of a third embodiment of the present systems for sampling and analyzing production fluid from an oil and gas well.
**FIG. 6** is a graph illustrating detection of a water portion of a sample of production fluid.
**FIG. 7** are graphs of absorbance vs. pH created using fluids having known pH and illustrating calibration of one or more sensors of some embodiments of the present systems and/or methods.
**FIG. 8** is a graph of absorbance vs. alkalinity created using fluids having known alkalinities and illustrating calibration of one or more sensors of some embodiments of the present systems and/or methods.
**FIG. 9** is a graph of absorbance vs. sulfate content created using fluids having known sulfate contents and illustrating calibration of one or more sensors of some embodiments of the present systems and/or methods.
**FIG. 10** is a graph of absorbance vs. strontium content created using fluids having known strontium contents and illustrating calibration of one or more sensors of some embodiments of the present systems and/or methods.
**FIG. 11** is graph of absorbance vs. total hardness created using fluids having known total hardness and illustrating calibration of one or more sensors of some embodiments of the present systems and/or methods.

### DETAILED DESCRIPTION

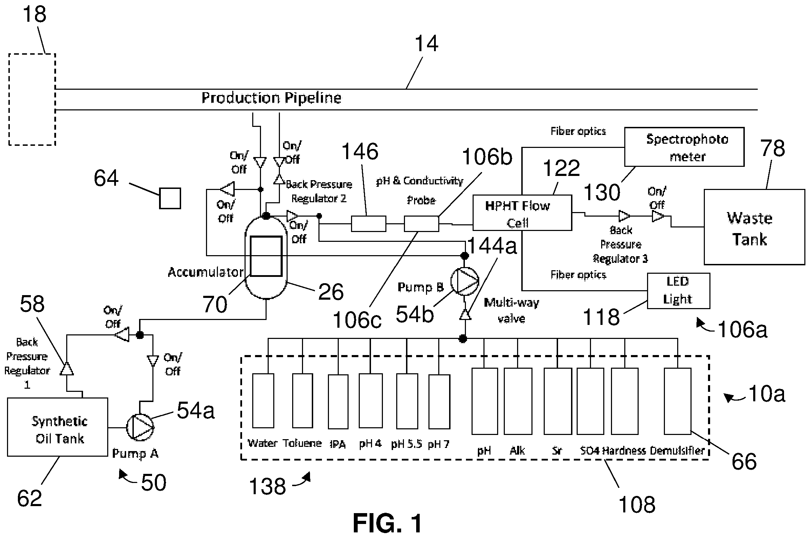

Referring to FIG. 1, shown is a first embodiment 10a of the present systems. As described in detail below, system 10a is configured to sample and/or analyze production fluid from an oil and gas well (*e.g.,* an onshore or offshore well). More particularly, system 10a can be coupled to and configured to receive a sample of production fluid from a production fluid conduit 14, which can comprise any suitable conduit through which production fluid is conveyed, such as, for example, production tubing, an annulus, a riser, a pipeline, and/or the like. System 10a can be coupled to such a conduit at any suitable location in a production system (*e.g.,* downhole, at a wellhead 18, downstream of the wellhead, or the like). Production fluid can be characterized as fluid that flows from a subterranean oil and gas reservoir and typically incudes a mixture of oil, gas, and water.

Such production fluid is provided by way of illustration, as the present systems can sample and/or analyze other fluids, including, for example, other hydrocarbon-containing fluids *(e.g.,* in upstream, midstream, and/or downstream processes), industrial process water *(e.g.,* boiler water, cooling tower water, waste water, and/or the like), and/or the like. A system for sampling and/or analyzing such a fluid can be coupled to and configured to receive a sample of that fluid from a conduit through which that fluid is conveyed; for example, if the fluid is industrial process water, the conduit can be a tube, pipe, other conduit and/or the like of a boiler, cooling tower, heat exchanger, condenser, pump, and/or the like.

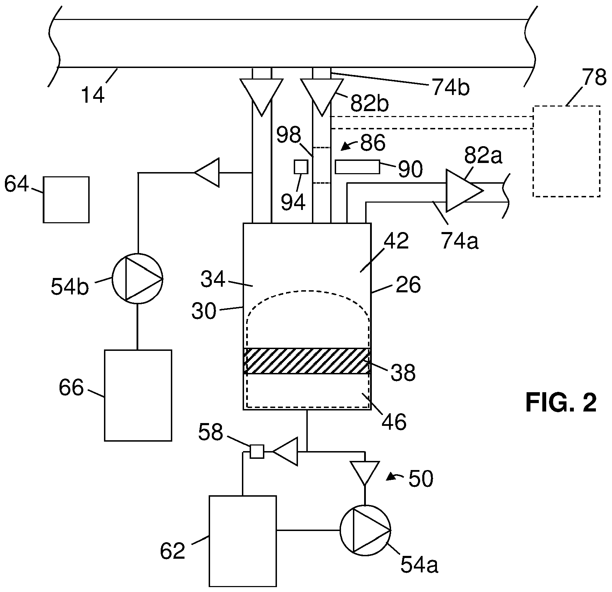

Referring additionally to FIG. 2, system 10a can include a container 26 for receiving a sample of production fluid from conduit 14. Provided by way of example, container 26 can include a housing 30 that defines an interior volume 34 and a divider 38 movably disposed within the housing and dividing the interior volume into a first chamber 42 and a second chamber 46. In system 10a, divider 38 comprises a piston; however, in other embodiments, a divider *(e.g.,* 38) of a container *(e.g.,* 26) can comprise a flexible bladder (*e.g.,* shown in dashed lines in FIG. 2). First chamber 42 can be placed into fluid communication with conduit 14, and, depending on pressure within the first chamber and/or second chamber 46, production fluid can be drawn from the conduit and into the first chamber. To illustrate, when first chamber 42 is in fluid communication with conduit 14 and pressure within second chamber 46 is lower than that within the first chamber, production fluid from the conduit can be drawn into the first chamber. Similarly, depending on pressure within first chamber 42 and second chamber 46, production fluid can be expelled from the first chamber. To illustrate, when pressure within second chamber 46 is higher than that within first chamber 42, production fluid can be expelled from the first chamber.

System 10a can include a pressure source 50 in fluid communication with second chamber 46 and configured to vary pressure within the second chamber. For example, pressure source 50 can comprise a pump 54a in fluid communication with second chamber 46. Pump 54a can comprise any suitable pump, such as, for example, a piston pump, gear pump, rotary vane pump, screw pump, and/or the like. In this embodiment, pressure source 50 can include a regulator 58 that, in conjunction with pump 54a, can be used to vary pressure within second chamber 46. Regulator 58 can mitigate pressure fluctuations caused by pump 54a, improve the response time of pressure source 50 to a desired change in pressure within second chamber 46, simplify control of the pressure source, and/or the like. Pressure source 50 can comprise a reservoir 62 configured to supply hydraulic fluid to pump 54a. Such hydraulic fluid can comprise any suitable hydraulic fluid, such as, for example, water, a water-based fluid, an oil-based fluid, and/or the like.

Container 26 can be configured to receive a sample of production fluid from conduit 14 such that a pressure of the received sample is substantially equal to a pressure of production fluid within the conduit. For example, prior to receiving a sample of production fluid from conduit 14 within first chamber 42, pressure within second chamber 46 can be greater than and/or substantially equal to a pressure of production fluid within the conduit and/or pressure within first chamber 42. To receive the sample of production fluid, pressure within second chamber 46 can be reduced (e.g., via control of pressure source 50): (1) to be substantially equal to, but less than, a pressure of production fluid within conduit 14 and/or pressure within first chamber 42; (2) such that a difference between a pressure of production fluid within the conduit and pressure within the second chamber and/or a difference between pressure within the first chamber and pressure within the second chamber is less than approximately 10 psi (*e.g.,* less than approximately 10, 9, 8, 7, 6, 5, 4, 3, 2, or 1 psi), in each instance, with pressure within the second chamber being the lesser of the two; and/or (3) pressure within the second chamber is less than but within 10% of (*e.g.,* within 10, 9, 8, 7, 6, 5, 4, 3, 2, or 1% of) pressure within the first chamber. In some instances, pressure within second chamber 46 can be maintained (e.g., via control of pressure source 50) such that one or more of the above relationships remains met as the sample is drawn into first chamber 42.

In system 10a, container 26 can be operated by a processor 64 in control of pressure source 50. And, such processor-based control can be facilitated by one or more sensors, such as, for example, pressure sensor(s) configured to capture data indicative of pressure within conduit 14, first chamber 42, second chamber 46, and/or the like, sensor(s) configured to capture data indicative of a position, velocity, and/or acceleration of divider 38 relative to housing 30, and/or the like. To illustrate, to receive a sample of production fluid from conduit 14 within the first chamber 42, and, in some instances, as the sample is received within the first chamber, processor 64 can control pressure source 50 such that pressure within second chamber 46, as indicated in data captured by one or more of the sensor(s), meets one or more of the above relationships. To further illustrate, processor 64 can be configured to control pressure source 50 such that, when receiving a sample of production fluid within first chamber 42 and/or expelling the sample from the first chamber, a position, velocity, and/or acceleration of divider 38 relative to housing 30, as indicated in data captured by one or more of the sensor(s), is less than, greater than, or equal to a target or threshold position, velocity, and/or acceleration.

System 10a can include a heating element 70 configured to heat container 26. Such a heating element can include, for example, heat tape, a heat blanket, an infrared heating element, another heating element, and/or the like, and heat transfer between the heating element and container 26 can be via conduction, convection (*e.g.,* in some instances, facilitated by one or more fans), and/or radiant heating. To reduce the amount of energy needed to heat container 26, the container can be insulated. Heating of container 26 can, for example, facilitate maintenance of a temperature of production fluid within the container at its temperature prior to being received by the container, separation of production fluid within the container, and/or the like.

Heating element 70 can be controlled in any suitable fashion. For example, system 10a can include one or more sensors configured to capture data indicative of a temperature of container 26 and/or production fluid within the container, and processor 64 can control-including activate, deactivate, increase power supplied to, and/or decrease power supplied to-heating element 70 based, at least in part, on data captured by the sensor(s). To illustrate, if data captured by the sensor(s) indicates that a temperature of container 26 and/or production fluid within the container is less than (e.g., more than a threshold amount less than) a target temperature, processor 64 can activate or increase power supplied to heating element 70, and, if data captured by the sensor(s) indicates that a temperature of the container and/or production fluid within the container is greater than (*e.g.,* more than a threshold amount greater than) a target temperature, processor 64 can deactivate or decrease power supplied to the heating element. Such a target temperature can be, for example, a temperature of production fluid within conduit 14 (*e.g.,* as indicated in data captured by one or more sensors), a commanded temperature, and/or the like.

Container 26 can be configured to receive a sample of production fluid from conduit 14 such that a temperature of the received sample is substantially equal to a temperature of production fluid within the conduit. Such functionality can be facilitated by, for example, heating element 70, coupling of container 26 to conduit 14, the container being in thermal communication with the conduit, the container receiving the sample of production fluid from the conduit such that a pressure of the received sample is substantially equal to a pressure of production fluid within the conduit, and/or the like. By receiving the sample of production fluid such that a temperature and/or pressure of the sample is substantially equal to a temperature and/or pressure of production fluid within conduit 14, system 10a can mitigate undesirable changes to propert(ies) of the sample that might otherwise occur when changing the temperature and/or pressure of the sample (*e.g.,* due to degassing, precipitation, and/or the like).

Some embodiments of the present methods for sampling and/or analyzing production fluid from an oil and gas well comprise receiving, into a container (*e.g.,* 26) coupled to a production fluid conduit (*e.g.,* 14), production fluid from the production fluid conduit. In some methods, the container comprises a housing (*e.g.,* 30) defining an interior volume *(e.g.,* 34) and a divider *(e.g.,* 38) movably disposed within the housing and dividing the interior volume into a first chamber *(e.g.,* 42) and a second chamber *(e.g.,* 46), wherein the receiving comprises reducing pressure within the second chamber to draw production fluid into the first chamber. In some methods, the divider comprises a piston or a flexible bladder. In some methods, the reducing pressure within the second chamber is performed using a pump *(e.g.,* 54a) and/or a regulator *(e.g.,* 58) in fluid communication with the second chamber.

In some methods, the received production fluid has a first pressure that is substantially equal to a pressure of production fluid within the production fluid conduit. In some methods, prior to the receiving, pressure within the second chamber is substantially equal to a pressure of production fluid within the production fluid conduit, and the reducing pressure within the second chamber comprises reducing pressure within the second chamber by between approximately 1 psi and approximately 10 psi. In some methods, the received production fluid has a first temperature that is substantially equal to a temperature of production fluid within the production fluid conduit.

System 10a can be configured to separate production fluid within container 26 into an oil and/or gas portion and a water portion having a higher water content than that of the oil and/or gas portion. To illustrate, the oil and/or gas portion can have a water content that is less than or substantially equal to any one of, or between any two of: 5, 10, 15, 20, 25, 30, 35, 40, 45 or more %, and the water portion can have a water content that is greater than or substantially equal to any one of, or between any two of: 50, 55, 60, 65, 70, 75, 80, 85, 90, 95, or more %. For example, system 10a can be configured to retain (*e.g.,* via processor 64 control of pressure source 50, valve(s), and/or the like) production fluid within container 26 for a period of time, such as, for example, a period of time that is sufficient to separate, via gravity separation, production fluid within the container into the oil and/or gas portion and the water portion, a period of time that is greater than or substantially equal to any one of, or between any two of: 1, 2, 3, 4, 5, 6, 7, 8, 9, 10, 12, 14, 16, 18, 20, 22, 24, 26, 28, 30, 35, 40, 45, 50, 55, 60, 70, 80, 90, 100, 110, 120, or more minutes (*e.g.,* between approximately 5 minutes and approximately 60 minutes), and/or the like. Such a period of time can be predetermined considering, for example, propert(ies) of production fluid within container 26, experimental and/or historical data that indicates a period of time sufficient to separate production fluid into an oil and/or gas portion and a water portion, and/or the like.

Such a period of time can be based, at least in part, on data captured by one or more sensors, such as, for example, sensor(s) configured to capture data indicative of a water content of a portion of production fluid within container 26. To illustrate, processor 64 can be configured to retain production fluid within container 26 until data captured by one or more of the sensor(s) indicates that a portion of production fluid within the container has a water content that is greater than, less than, or equal to a target or threshold water content. More specifically, processor 64 can be configured to retain production fluid within the container until data captured by one or more of the sensor(s) indicates that a portion of the production fluid at or proximate to the lower end of container 26 (*e.g.,* a lower end of a first chamber 42 of the container) is greater than or equal to a target or threshold water content, a portion of the production fluid at or proximate to the upper end of the container (*e.g.,* an upper end of the first chamber) is less than or equal to a target or threshold water content, and/or the like.

For further example, system 10a can be configured to provide a demulsifier to production fluid within container 26. For example, system 10a can include a reservoir 66 within which the demulsifier can be disposed, and a pump 54b configured to be in fluid communication between the reservoir and first chamber 42 of container 26 such that actuation of the pump (e.g., which can be controlled by processor 64) provides the demulsifier from the reservoir and to the first chamber. Pump 54b can comprise any suitable pump, such as, for example, any pump described above. Such a demulsifier can include any suitable demulsifier, such as, for example, an acid catalyzed phenol-formaldehyde resin, a base catalyzed phenol-formaldehyde resin, an epoxy resin, a polyethyleneimine, a polyamine, a di-epoxide, a polyol, dendrimer, or a combination thereof, which can be ethoxylated and/or propoxylated.

In system 10a, separation of production fluid within container 26 into the oil and/or gas portion and the water portion can be facilitated via movement of divider 38 relative to housing 30. For example, divider 38 can be reciprocated relative to housing 30 to disturb production fluid within container 26, which can facilitate mixture of a demulsifier (if present) with the production fluid, separation of gas from other portions of the production fluid, and/or the like. Such reciprocation of divider 38 relative to housing 30 can be performed at any suitable amplitude, whether constant or varying, and at any suitable frequency, whether constant or varying.

Such separation can, in some instances, be facilitated by heating container 26 (*e.g.,* using heating element 70). To illustrate, in such instances, container 26 can be heated to a temperature that is greater than or substantially equal to any one of, or between any two of: 50, 55, 60, 65, 70, 75, 80, 85, 90, or 95 degrees Celsius (*e.g.,* approximately 70 or 90 degrees Celsius).

Some embodiments of the present methods comprise separating, within a container *(e.g.,* 26), production fluid into an oil and/or gas portion and a water portion having a higher water content than that of the oil and/or gas portion. In some methods, the separating comprises retaining the production fluid within the container for a period of time. In some methods, the period of time is between approximately 5 minutes and approximately 60 minutes. In some methods, the separating comprises providing a demulsifier to the production fluid.

In some methods, fluid received by a container (*e.g.,* 26) may be separated-in lieu of or in addition to within the container-by directing the received fluid to one or more separators, other containers, and/or the like disposed downstream of the container. In some methods, separation of fluid received by a container (*e.g.,* 26) may not be necessary and/or may be undesirable, such as, for example, when analyzing certain properties of certain fluids *(e.g.,* certain properties of industrial process water).

System 10a can be configured to direct production fluid within container 26 to one or more locations (*e.g.,* for analysis, removal from the system, disposal, and/or the like). To mitigate undesirable changes to its pressure, as at least a portion of production fluid within container 26-such as the portion to be directed to one or more sensors, described below-is expelled from the container, pressure within second chamber 46 can *(e.g.,* via control of pressure source 50) be limited: (1) to one that is substantially equal to, but greater than, pressure within first chamber 42; (2) such that a difference between pressure within the first chamber and pressure within the second chamber is less than approximately 10 psi (*e.g.,* less than approximately 10, 9, 8, 7, 6, 5, 4, 3, 2, or 1 psi), with pressure within the second chamber being the greater of the two; and/or (3) pressure within the second chamber is greater than but within 10% of (*e.g.,* within 10, 9, 8, 7, 6, 5, 4, 3, 2, or 1% of) pressure within the first chamber. Similarly to as described above, such can control can be facilitated by processor 64, which, using data captured by one or more sensors, can control pressure source 50 such that pressure within second chamber 46 meets one or more of the above relationships.

System 10a can include a first conduit 74a in fluid communication with container 26 *(e.g.,* first chamber 42 thereof) and configured to convey production fluid from the container to one or more sensors (*e.g.,* 106a, 106b, 106c, and/or the like, described below). For further example, system 10a can include a second conduit 74b in fluid communication with container 26 and configured to convey production fluid from the container to conduit 14 and/or to a reservoir 78 (*e.g.,* for removal from the system and/or disposal). System 10a can include one or more valves (*e.g.,* 82a, 82b, and/or the like, which can be controlled by processor 64) for controlling fluid communication through first conduit 74a and/or second conduit 74b and thus the location(s) to which production fluid within container 26 is directed.

More particularly, system 10a can be configured to direct at least a portion of production fluid within container 26, such as at least a portion of the water portion, to one or more sensors (e.g., 106a, 106b, 106c, and/or the like) and/or at least a portion of the production fluid, such as at least a portion of the oil and/or gas portion, to conduit 14 and/or reservoir 78. For example, system 10a can include a sensor 86 configured to capture data indicative of a water content of at least a portion of production fluid within and/or flowing from container 26. If data captured by sensor 86 indicates that the portion of the production fluid has a water content that is greater than or equal to a target or threshold water content, production fluid within container 26 can be directed (e.g., via control of valve(s) 82a, 82b, and/or the like) through conduit 74a. Conversely, if data captured by sensor 86 indicates that the portion of the production fluid has a water content that is less than or equal to a target or threshold water content, production fluid within container 26 can be directed through conduit 74b.

Sensor 86 can comprise an optical sensor. For example, sensor 86 can include a light source 90, such as, for example, a laser, and a detector 94. During use of sensor 86, light from light source 90 can be passed through at least a portion of production fluid within and/or flowing from container 26 before reaching detector 94. As shown in more detail in Example 1, at least because oil may interact with light from light source 90 differently than water (*e.g.,* oil may be more opaque, absorb more light, and/or the like than water), the intensity and/or other characteristic(s) of light from the light source that reaches detector 94 can be indicative of a water content of the portion of the production fluid.

In this embodiment, sensor 86 is coupled to conduit 74b. More particularly, sensor 86 can be coupled to conduit 74b such that light source 90 and detector 94 are not exposed to production fluid within the conduit. For example, light source 90 and detector 94 can be disposed exteriorly to conduit 74b, and the conduit can include a transparent or translucent section 98 through which light can pass between light source 90 and detector 94. Transparent or translucent section 98 can comprise glass, a polymer (*e.g.,* perfluoroalkoxy, polytetrafluoroethylene, fluorinated ethylene propylene, and/or the like), and/or the like. In other embodiments, a sensor (*e.g.,* 86) can be at least partially disposed within a conduit (*e.g.,* 74b), coupled to a container (*e.g.,* 26), whether disposed exteriorly to the container *(e.g.,* the container can include a transparent or translucent section) or at least partially disposed within the container, and/or the like. Sensor 86 is provided only by way of example, as other embodiments can comprise any suitable sensor (*e.g.,* 86), such as, for example, a sensor configured to capture data indicative of a pressure and/or flow rate of production fluid within a container (*e.g.,* 26), a conduit *(e.g.,* 74a, 74b, and/or the like), and/or the like *(e.g.,* the pressure and/or flow rate of the production fluid may vary depending on the water content of the production fluid), a sensor configured to capture data indicative of a pressure and/or flow rate of hydraulic fluid within the container, a pressure source (*e.g.,* 50), and/or the like, and/or the like.

To illustrate, in system 10a, production fluid from container 26 can be directed through second conduit 74b until data captured by sensor 86 indicates that a water content of production fluid within the second conduit meets or exceeds a threshold or target water content, after which one or more valves (e.g., 82a, 82b, and/or the like) can be actuated to block fluid communication through the second conduit and allow fluid communication through first conduit 74a, thereby directing production fluid from the container through the first conduit.

Some embodiments of the present methods comprise directing at least a first portion of production fluid within a container (*e.g.,* 26), such as an oil and/or gas portion of the production fluid, to at least one of a production fluid conduit *(e.g.,* 14) and a reservoir *(e.g.,* 78) and at least a second portion of the production fluid, such as a water portion of the production fluid, to one or more sensors (*e.g.,* 106a, 106b, 106c, and/or the like), wherein the directing is performed based, at least in part, on data captured by a sensor (*e.g.,* 86), the data being indicative of a water content of the first portion and/or the second portion. In some methods, the sensor comprises an optical sensor.

Container 26 is provided by way of illustration, as other embodiments of the present systems can comprise any suitable container for receiving a sample of fluid from a conduit *(e.g.,* 14). For example, in some embodiments, a container can include a first chamber *(e.g.,* 42) and a second chamber *(e.g.,* 46) defined within separate housings, within a same housing but remote from one another, and/or the like. In such a container, a divider *(e.g.,* 38) can include, for each of the first and second chambers, a piston for varying the volume of the chamber, where the pistons are mechanically coupled to one another (e.g., via a rod). In some embodiments, a container can include a piston *(e.g.,* 38) that is mechanically coupled to *(e.g.,* via a rod) and movable by an actuator-such a container may not include a second chamber *(e.g.,* 46). In some embodiments, a container may have a fixed volume.

For further example, in some embodiments, a container (*e.g.,* 26) can include a divider *(e.g.,* 38) having an area on which pressure within a first chamber *(e.g.,* 42) of the container acts ("first area") that differs from an area of the divider on which pressure within a second chamber *(e.g.,* 46) of the container acts ("second area"). In such embodiments, the container can be controlled taking into account such differing areas, but otherwise similarly to as described above.

To illustrate, in these embodiments, to receive production fluid within the first chamber, pressure within the second chamber can be reduced *(e.g.,* via control of a pressure source 50 in fluid communication with the second chamber): (1) to be substantially equal to, but less than, pressure within the first chamber times the first area and divided by the second area; (2) such that a difference between pressure within the second chamber times the second area and pressure within the first chamber times the first area is less than approximately 10 psi times the second area *(e.g.,* less than approximately 10, 9, 8, 7, 6, 5, 4, 3, 2, or 1 psi times the second area), with the former value being the lesser of the two; and/or (3) pressure within the second chamber times the second area-equal to a force acting on the divider due to pressure within the second chamber-is less than but within 10% of (*e.g.,* within 10, 9, 8, 7, 6, 5, 4, 3, 2, or 1% of) pressure within the first chamber times the first area-equal to a force acting on the divider due to pressure within the first chamber.

To further illustrate, in these embodiments, as at least a portion of production fluid within the container is expelled from the container, pressure within the second chamber can be (*e.g.,* via control of the pressure source) limited: (1) to one that is substantially equal to, but greater than, pressure within the first chamber times the first area and divided by the second area; (2) such that a difference between pressure within the second chamber times the second area and pressure within the first chamber times the first area is less than approximately 10 psi times the second area (e.g., less than approximately 10, 9, 8, 7, 6, 5, 4, 3, 2, or 1 psi times the second area), with the former value being the greater of the two; and/or (3) pressure within the second chamber times the second area is greater than but within 10% of (*e.g.,* within 10, 9, 8, 7, 6, 5, 4, 3, 2, or 1% of) pressure within the first chamber times the first area.

System 10a can include one or more sensors configured to capture data indicative of one or more properties of production fluid received by container 26, and more particularly, the water portion of the production fluid. Such propert(ies) can include any suitable property, such as, for example, a TDS content, a pH, an alkalinity, a total hardness, a hydrogen sulfide content, an ammonia content, a hydrocarbon content, a carbon dioxide content, a total metal carbonate content, a total metal sulfate content, a total metals content, a sodium chloride content, a silicate content, an iron content, a calcium content, a sodium content, a magnesium content, a potassium content, a strontium content, a chlorine content, a chloride content, a bicarbonate content, a phosphorous content, a boron content, a barium content, a sulfate content, an iron content, a nickel content, a chromium content, a cobalt content, a molybdenum content, a specific gravity, a conductivity, a saturation index and/or ratio, a resistivity, a pressure, and/or a temperature. Such sensor(s) can include any suitable sensor, such as, for example, a spectrophotometer, a pH probe 106b, a conductivity probe 106c, an ion-selective electrode, a temperature sensor, a pressure sensor, a flow sensor, a viscometer, and/or the like.

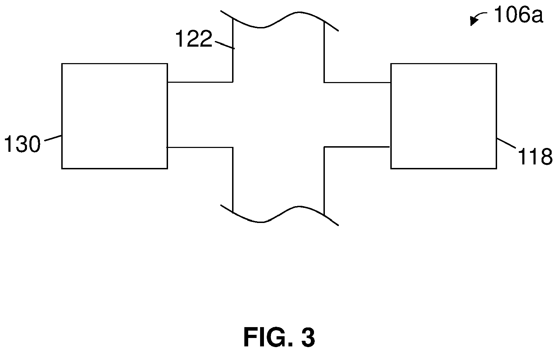

For example, and referring additionally to FIG. 3, shown is a sensor 106a that may be suitable for use in some embodiments (e.g., 10a) of the present systems. Sensor 106a can include a flow cell 122 through which production fluid can be conveyed. Sensor 106a can include a light source 118, such as, for example, a light-emitting diode light source, and a detector 130. Detector 130 can comprise a spectrophotometer (*e.g.,* a photometer that is capable of measuring light intensity as a function of wavelength). During use of sensor 106a, production fluid can be conveyed through flow cell 122, and light from light source 118 can be directed through the production fluid within the flow cell and to detector 130. The intensity and/or other characteristic(s) of light from light source 118 that reaches detector 130 can be indicative of propert(ies) of the production fluid.

In some instances, before production fluid is analyzed using sensor(s) (*e.g.,* 106a, 106b, 106c, and/or the like), one or more additives, such as, for example, diluent(s), reagent(s), solvent(s), and/or the like, can be provided to the production fluid. For example, system 10a can include one or more reservoirs 138 for storing the additive(s), and the additive(s) can be provided from the reservoir(s) and to the production fluid using pump 54b. To allow additive(s) in one or more of reservoirs 138 to be provided to the production fluid independently of additive(s) in other(s) of the reservoirs, system 10a can include a multi-way valve 144a disposed between the reservoirs and pump 54b. System 10a can include a mixer 146, such as, for example, an in-line mixer, configured to mix the additive(s) with the production fluid. Such additive(s) can be provided to the production fluid within container 26, within first conduit 74a, downstream of the first conduit, and/or the like.

For example, at least one of the additive(s) can comprise a reagent that is responsive to pH and/or alkalinity, non-limiting examples of which include thymol blue, methyl red, bromothymol blue, bromocresol green, bromocresol purple, and/or phenolphthalein. For further example, at least one of the additive(s) can comprise a reagent that is responsive to iron, non-limiting examples of which include 1,10-phenanthroline, 4,7-diphenyl-l,IO-phenanthroline, 2,4,6-tris(2-pyridyl)-1,3,5triazine, 2,2 bypyridine, potassium cyanide, and/or 2,2',2" tripyridine. For yet further example, at least one of the additive(s) can comprise a chelating agent, non-limiting examples of which include ethylenediaminetetraacetic acid (EDTA), nitrilotriacetic acid (NTA), disuccinic acid, glucoheptonate, monoethanolethylenediamine triacetic acid, diethylenatriamine pentacetic acid, and/or citric acid.

Some additives, such as some color reagents, may not function properly at elevated temperatures and/or may perform differently at different temperatures, each of which may frustrate obtaining consistent sensor data using such additives. To mitigate these issues, system 10a can include a cooling system 108 configured to control a temperature of one or more of reservoir(s) 138 (*e.g.,* at least the reservoir(s) within which such additives are disposed). To illustrate, cooling system 108 can maintain such reservoir(s) at a temperature that is less than or substantially equal to any one of, or between any two of: 15, 20, 25, 30, 35, or 40 degrees Celsius. Cooling system 108 can comprise any suitable cooling system, such as, for example, an air conditioner (*e.g.,* a 5000 British thermal unit per hour air conditioner).

Measurement of some properties, such as pH, conductivity, and sulfate content, may be temperature-dependent; thus, some systems can include a cooling system (*e.g.,* 108) configured to control a temperature of portion(s) of the system at which data indicative of such propert(ies) is captured, including, for example, a sensor (*e.g.,* pH probe 106b, conductivity probe 106b, and/or sensor 106a) for collecting data indicative of such a property and/or a portion of a conduit that directs production fluid through that sensor. To illustrate, the cooling system can maintain such portion(s) at a temperature that is less than or substantially equal to any one of, or between any two of: 15, 20, 25, 30, 35, or 40 degrees Celsius.

One or more sensors (*e.g.,* 106a, 106b, 106c, and/or the like) can be calibrated. For example, a sensor can be used to capture data indicative of a property of a fluid for which the property is known, thereby providing a correlation between data captured by the sensor and the property. That correlation can then be applied to subsequent data captured by the sensor indicative of the property of a fluid for which the property is unknown (*e.g.,* production fluid) to thereby determine that previously-unknown property. Examples of such correlations are included in Examples 2-6, below. Fluid(s) for use in such calibration can comprise any suitable fluid, such as, for example, a stock solution and/or a diluent (*e.g.,* water, an alcohol, a glycol, a mineral acid, an organic acid, a buffer, ammonium hydroxide, and/or the like). Such fluid(s) can be stored in reservoir(s) 138 and can be provided to sensor(s) using a pump (*e.g.,* pump 54b). At least via including such fluid(s), system 10a can allow for periodic calibration of sensor(s). Particularly when using reagents in conjunction with sensor(s), such periodic calibration can facilitate accurate measurements from the sensor(s) (*e.g.,* certain reagents may have characteristic(s) that change over time).

Some embodiments of the present methods comprise capturing, with one or more sensors (*e.g.,* 106a, 106b, 106c, and/or the like), data indicative of one or more properties of a water portion of production fluid received by a container (*e.g.,* 26). In some methods, the water portion, during the capturing, has a pressure that is substantially equal to a pressure of production fluid within a production fluid conduit (*e.g.,* 18) that is coupled to the container. In some methods, the water portion, during the capturing, has a temperature that is substantially equal to a temperature of production fluid within the production fluid conduit. In some methods, the one or more properties comprise any one or more of the propert(ies) described above. In some methods, the one or more sensors comprises a spectrophotometer *(e.g.,* 106a). In some methods, the one or more sensors comprises a pH probe *(e.g.,* 106b). In some methods, the one or more sensors comprises a conductivity probe (*e.g.,* 106c). In some methods, the one or more sensors comprises an ion-selective electrode.

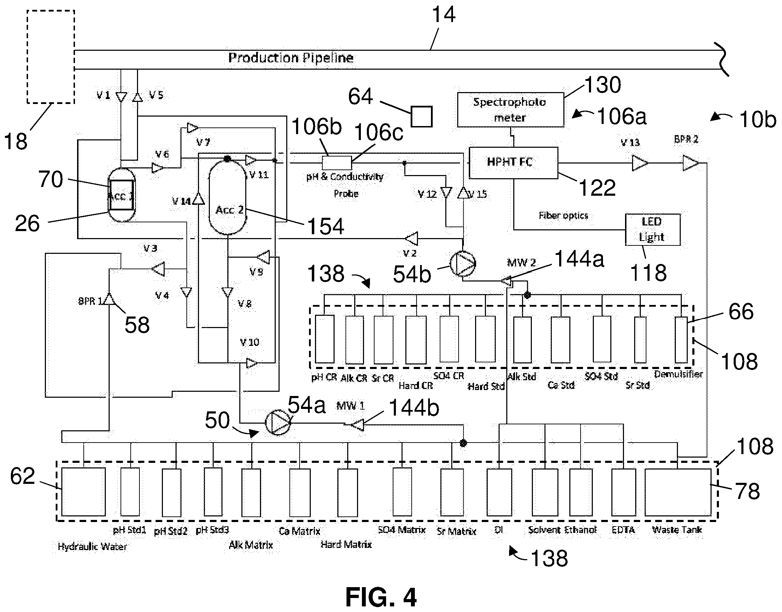

Referring now to FIG. 4, shown is a second embodiment 10b of the present systems. System 10b can be substantially similar to system 10a, with the primary exceptions described below. System 10b can include a container 154 configured to receive and facilitate mixing of production fluid and additive(s), demulsifier(s), and/or the like and/or to receive and facilitate mixing of stock solution(s) and/or diluent(s). In at least this way, system 10b may not include a mixer 146. Container 154 can have a fixed or variable volume (*e.g.,* the container can comprise an accumulator).

In system 10b, composition(s)-additive(s), demulsifier(s), stock solution(s), and/or diluent(s)-from one or more of reservoirs 138 can be provided via pump 54b (*e.g.,* facilitated using multi-way valve 144a), and composition(s) from other(s) of the reservoirs can be provided via pump 54a (*e.g.,* facilitated using multi-way valve 144b). In this way, compositions from reservoirs 138 can be introduced to different portions of system 10b; for example, one(s) provided by pump 54a can be introduced to container 154, and one(s) provided by pump 54b can be introduced to a portion of system 10b downstream of container 154.

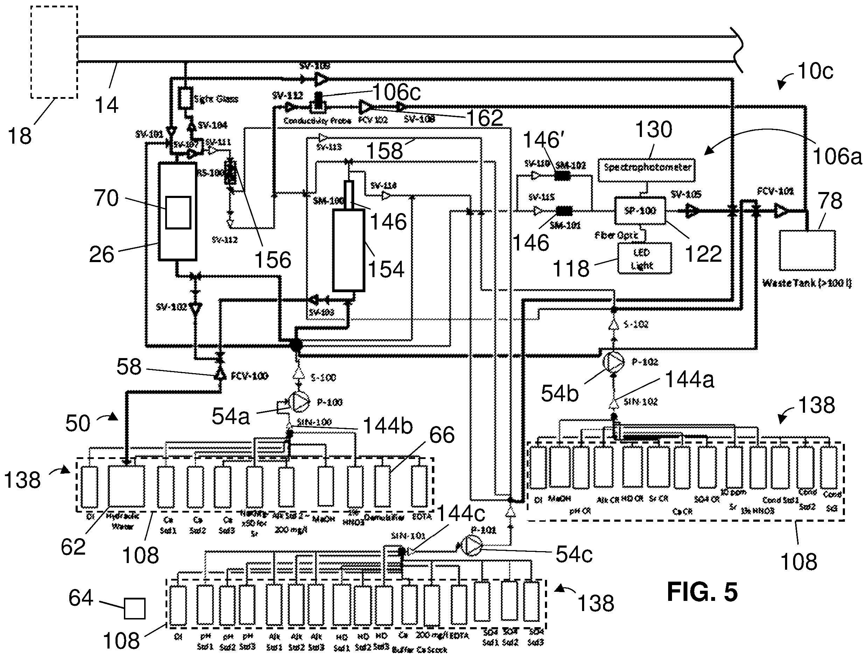

Referring now to FIG. 5, shown is a third embodiment 10c of the present systems. System 10c can be substantially similar to system 10b, with the primary exceptions described below. In addition to pump 54b and multi-way valve 144a and pump 54a and multi-way valve 144b, system 10c includes a pump 54c and a multi-way valve 144c for providing composition(s) from one or more reservoirs 138; thus, system 10c has increased flexibility for introducing compositions from reservoirs 138 to different portions of the system.

As shown, system 10c includes a filter 156 for removing oil from fluid that flows from container 26 and to sensor(s) (*e.g.,* 106a, 106b, 106c, and/or the like) for analyzing the fluid. Such a filter can mitigate the presence of oil in fluid at the sensor(s), thereby promoting accurate measurements by the sensor(s). For similar reasons, system 10c can include a conduit 158 for flushing container 154, filter 156, container 26, and/or the like (*e.g.,* with deionized water provided by pump 54c).

Conductivity probe 106c can be positioned to capture data indicative of fluid flowing through a pressure-regulated (*e.g.,* via regulator 162) conduit. Through use of this pressure-regulated conduit, fluid flowing past conductivity probe 106c may be at a lower pressure than fluid in other portions of the system; in this way, the conductivity probe may be a lower-cost conductivity probe. In instances when sulfate measurements are performed, an independent line and mixer (*e.g.,* 146') can be used to minimize cross-contamination by the sulfate to other elements flowing through mixer 146.

### EXAMPLES

The present invention will be described in greater detail by way of specific examples. The following examples are offered for illustrative purposes only, and are not intended to limit the invention in any manner. Those of skill in the art will readily recognize a variety of noncritical parameters which can be changed or modified to yield essentially the same results.

### EXAMPLE 1

### (Optical Sensor-Based Water Detection)

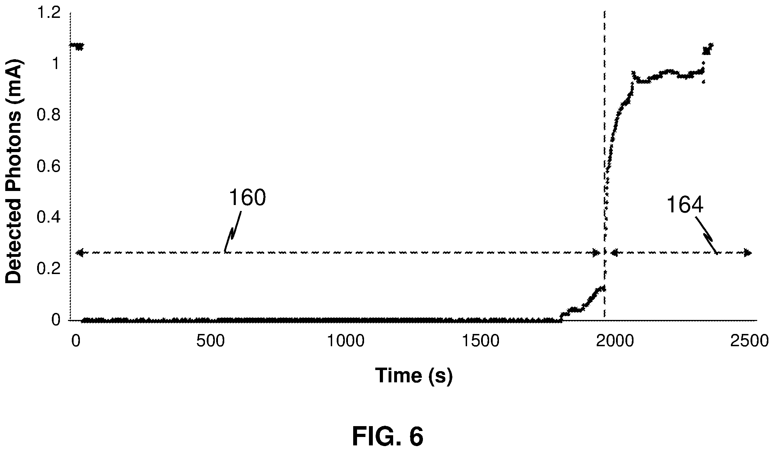

Production fluid within a container (*e.g.,* 26) was separated into a water portion and an oil and/or gas portion. Production fluid from the container was then directed through a conduit *(e.g.,* 74b) having a transparent or translucent section (*e.g.,* 98). A sensor (*e.g.,* 86) having a laser *(e.g.,* 90) and a detector *(e.g.,* 94) was positioned such that light from the laser travelled through the transparent or translucent section before reaching the detector. Referring to FIG. 6, as the oil and/or gas portion flowed through the transparent or translucent section (*e.g.,* during time period 160), the sensor detected a relatively small number of photons (measured in milliamps) as compared to when the water portion flowed through the transparent or translucent section (*e.g.,* during time period 164).

### EXAMPLE 2

### (Exemplary Reagents used with the System of the Present Invention)

Examples 2-6 include exemplary reagents used in the system of Example 1. As some reagents are sensitive to oxygen and/or light, the exemplary reagents discussed below were prepared under non-oxygen atmospheres and were shielded from light sources.

### pH Color Reagent and Stock Solution Compositions and pH Spectrophotometric Measurements

### A. pH Color Reagent Compositions

An exemplary pH color reagent was prepared as follows. First, dissolve 0.05 grams (g) of neutral red in 100 milliliters (mL) of methanol, 0.5 g of methyl red in 100 ml of methanol, and 0.5 g of methyl yellow in 100 ml of methanol to make three dye solutions. Stir each dye solution at 600 revolutions per minute (RPM) for 30 minutes and filter each dye solution using a 0.45 micrometer (µm) filter. Next, produce a first solution comprising 50.8 mL of the neutral red dye solution, 20.4 mL of the methyl red dye solution, 15.6 mL of the methyl yellow dye solution, and 413.2 mL of methanol. Finally, produce a second solution by taking 50 mL of the first solution and adding 450 mL of deionized water.

### B. pH Stock Solution Compositions

An exemplary pH stock solution was prepared by dissolving 5 mM PIPES buffer in synthetic brine 1 or brine 2 (including only Na, K, Mg, Ca, Sr, and Ba salts) and adjusting the pH of the solution (e.g., to 4.0, 5.0, 5.5, 6.0, or 7.0) by adding 1 M HCl.

### C. pH Spectrophotometric Measurements

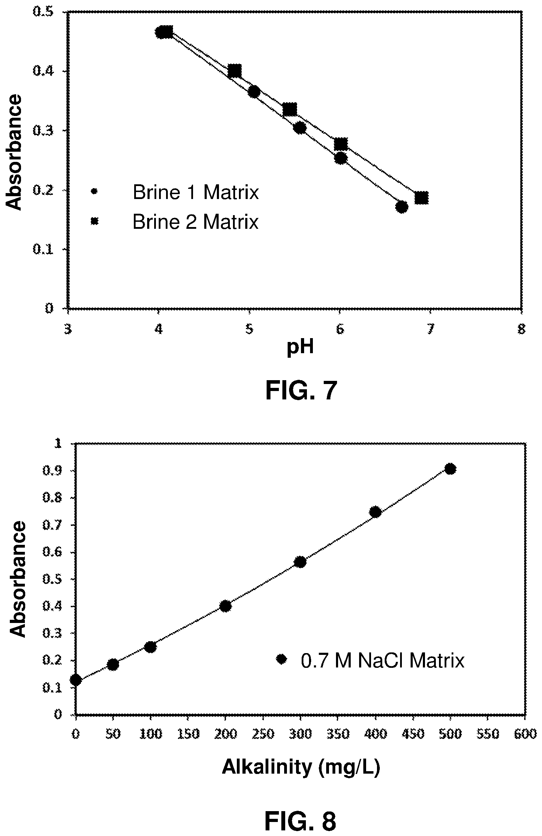

Mixed 8 mL of a pH color reagent with 2 mL of a pH stock solution (*e.g.,* when calibrating the spectrophotometer) or production fluid (*e.g.,* when analyzing the production fluid with the spectrophotometer). Data captured by the spectrophotometer at 555 nanometers (nm). FIG. 7 shows a graph of absorbance vs. pH created using pH stock solutions having known pH.

### EXAMPLE 3

### (Alkalinity Color Reagent and Stock Solution Compositions and Alkalinity Spectrophotometric Measurements)

### A. Alkalinity Color Reagent Compositions

An exemplary alkalinity color reagent was prepared as follows. First, dissolve 1.352 g of bromocresol green sodium salt in 100 mL of methanol to form a dye solution. Stir the dye solution at 600 RPM for 30 minutes and filter the dye solution with a 0.45 µm filter. Next, prepare 2 liters (L) of a buffer solution, the buffer solution comprising 11.809 g of succinic acid, 1.0206 g of sodium acetate, 82 g of NaCl, and 5.29 mL 100% acetic acid, with the remainder comprising deionized water. Add 50 mL of the dye solution to 1950 mL of the buffer solution. Finally, add 1.6 mL of 10 M NaOH to the dye and buffer solution to adjust the pH of the dye and buffer solution to 3.2.

### B. Alkalinity Stock Solution Compositions

Exemplary alkalinity stock solutions were prepared in 0.7 M NaCl matrix.

### C. Alkalinity Spectrophotometric Measurements

Mixed 9 mL of an alkalinity color reagent with 1 mL of an alkalinity stock solution *(e.g.,* when calibrating the spectrophotometer) or production fluid *(e.g.,* when analyzing the production fluid with the spectrophotometer). Data captured by the spectrophotometer at 617 nm. FIG. 8 shows a graph of absorbance vs. alkalinity created using alkalinity stock solutions having known alkalinities.

### EXAMPLE 4

### (Sulfate Content Color Reagent and Stock Solution Compositions and Sulfate Content Spectrophotometric Measurements)

### A. Sulfate Content Color Reagent Compositions

An exemplary sulfate content color reagent was prepared as follows:
(1) produce a solution by dissolving 71 g of BaCl₂·H₂0 in 300 mL of deionized water;
(2) dissolve 20 g of NaCl in the solution;
(3) add 13 mL of 35% HCl dropwise to the solution;
(4) add 60 mL of isopropanol dropwise to the solution;
(5) add 100 mL of deionized water to the solution;
(6) add 50 mL of glycerol dropwise to the solution;
(7) stir the solution at 600 RPM for 30 minutes; and
(8) filter the solution using a 0.45 µm filter.

### B. Sulfate Content Stock Solution Compositions

Exemplary sulfate content stock solutions were prepared by diluting brine 1 or brine 2 and adding a known sulfate to the diluted brine.

### C. Sulfate Content Spectrophotometric Measurements

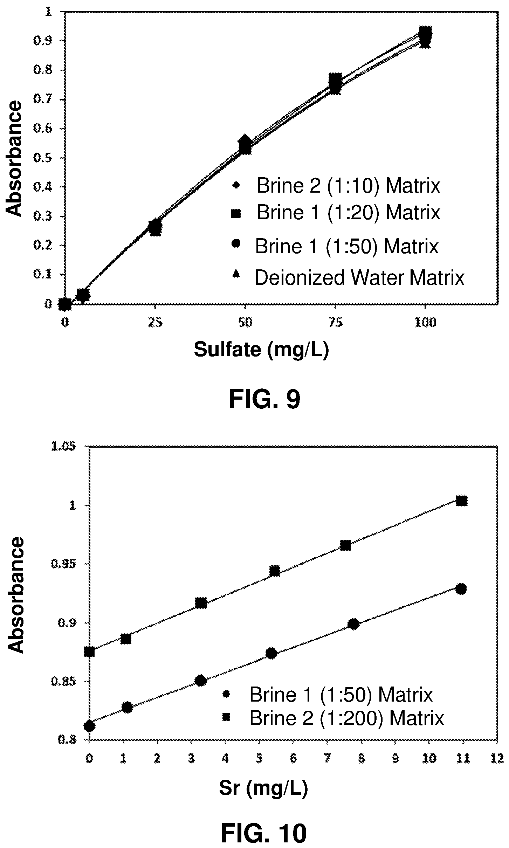

Mixed 0.5 mL of a sulfate content color reagent with 10 mL of a sulfate content stock solution (*e.g.,* when calibrating the spectrophotometer) or production fluid *(e.g.,* when analyzing the production fluid with the spectrophotometer). Data captured by the spectrophotometer at 420 nm. FIG. 9 shows a graph of absorbance vs. sulfate content created using sulfate content stock solutions having known sulfate contents.

### EXAMPLE 5

### (Strontium Content Color Reagent and Stock Solution Compositions and Strontium Content Spectrophotometric Measurements)

### A. Strontium Content Color Reagent Compositions

An exemplary strontium content color reagent was prepared as follows. Produce a first solution by dissolving 0.3106 g of sulfonazo III in 1 L of deionized water. Stir the first solution at 600 RPM for 30 minutes and filter the first solution using a 0.45 µm filter. Produce a second solution by dissolving 5.4 g of NaOH, 7.6 g of EGTA, and 7.0 g of maleic acid in 2 L of deionized water. Produce a third solution by mixing 200 mL of the second solution with 800 mL of deionized water. Finally, mix the first and third solutions.

### B. Strontium Content Stock Solution Compositions

Exemplary strontium content stock solutions was prepared by diluting brine 1 or brine 2 (without strontium) and adding a known amount of strontium to the diluted brine.

### C. Strontium Content Spectrophotometric Measurements

Mixed 2 mL of a strontium content color reagent with 2 mL of a strontium content stock solution (*e.g.,* when calibrating the spectrophotometer) or production fluid *(e.g.,* when analyzing the production fluid with the spectrophotometer). Data captured by the spectrophotometer at 643 nm. FIG. 10 shows a graph of absorbance vs. strontium content created using strontium content stock solutions having known strontium contents.

### EXAMPLE 6

### (Total Hardness Color Reagent and Stock Solution Compositions and Total Hardness Spectrophotometric Measurements)

### A. Total Hardness Color Reagent Compositions

An exemplary total hardness color reagent was prepared as follows. Prepare a buffer solution by dissolving 67.6 g NH₄Cl in 572 mL of 1 M NH₄OH and adding deionized water until the buffer solution is 1 L. Prepare an indicator solution by dissolving 0.25 g of calmagite in 500 mL of deionized water. Stir the indicator solution at 600 RPM for 30 minutes and filter the indicator solution using a 0.45 µm filter. Finally, mix 160 mL of the buffer solution, 32 mL of the indicator solution, 160 mL of 200 milligram per milliliter (mg/mL) MgEDTA, and 250 mL of deionized water.

### B. Total Hardness Stock Solution Compositions

Exemplary total hardness stock solutions were prepared by diluting brine 1 (with only NaCl and KCl salts) and adding 1,000 mg/L Ca (*e.g.,* 1 mg/L Ca is equivalent to 2.5 mg/L total hardness).

### C. Total Hardness Spectrophotometric Measurements

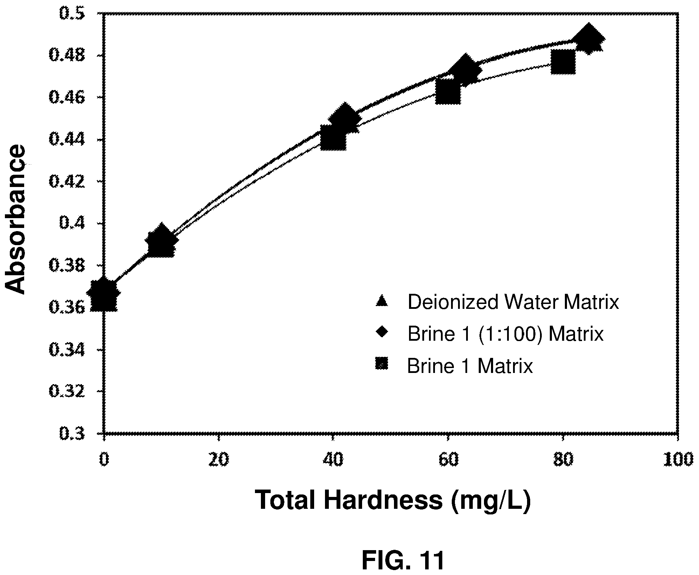

Mixed 6 mL of a total hardness color reagent with 0.8 mL of a total hardness stock solution (*e.g.,* when calibrating the spectrophotometer) or production fluid *(e.g.,* when analyzing the production fluid with the spectrophotometer). Data captured by the spectrophotometer at 520 nm. FIG. 11 shows a graph of absorbance vs. total hardness created using total hardness stock solutions having known total hardnesses.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiment. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. A method for sampling and analyzing production fluid from an oil and gas well, the method comprising:
receiving, into a first chamber (42) of a container (26) coupled to a production fluid conduit (14), production fluid from the production fluid conduit (14), the container (26) having:
a second chamber (46); and
a movable divider (38) disposed between and in fluid communication with each of the first chamber (42) and the second chamber (46);
wherein receiving production fluid into the first chamber (42) comprises drawing production fluid into the first chamber (42) at least by controlling a pressure source (50) in fluid communication with the second chamber (46) such that a force acting on the divider (38) due to pressure within the second chamber (46) is reduced to, and
remains as production fluid is drawn into the first chamber (42),
the method being **characterized in that**
the force acting on the divider is less than but within 10% of a force acting on the divider (38) due to pressure within the first chamber (42); and wherein the method further comprises:
expelling at least a portion of the received production fluid from the first chamber (42) at least by controlling the pressure source (50) such that a force acting on the divider (38) due to pressure within the second chamber (46) remains within 10% of a force acting on the divider (38) due to pressure within the first chamber (42);
directing at least a first portion of the expelled production fluid to one or more sensors (106a, 106b, 106c); and
capturing, with the one or more sensors (106a, 106b, 106c), data indicative of one or more properties of at least a portion of the received production fluid.

2. The method of claim 1, comprising:
directing at least a second portion of the expelled production fluid to at least one of the production fluid conduit (14) and a reservoir (78);
wherein directing the first and second portions is performed based, at least in part, on data captured by a sensor (86) that is indicative of a water content of the first portion and/or the second portion.

3. The method of claim 1, comprising calibrating at least one of the one or more sensors (106a, 106b, 106c) at least by capturing, with the at least one sensor, data indicative of one or more properties of a fluid, wherein at least one of the one or more properties of the fluid is known.

4. The method of claim 1, comprising providing one or more reagents to the portion of the received production fluid (14).

5. The method of claim 4, wherein at least one of the one or more reagents is responsive to pH and/or alkalinity and comprises thymol blue, methyl red, bromothymol blue, bromocresol green, bromocresol purple, and/or phenolphthalein, or wherein at least one of the one or more reagents is responsive to iron and comprises 1,10-phenanthroline, 4,7-diphenyl-l,IO-phenanthroline, 2,4,6-tris(2-pyridyl)-l,3,5triazine, 2,2 bypyridine, potassium cyanide, and/or 2,2',2" tripyridine, or wherein at least one of the one or more reagents comprises a chelating agent.

6. A system (10a) for sampling and analyzing production fluid from an oil and gas well, the system (10a) comprising:
a container (26) having:
a first chamber (42) configured to be in fluid communication with a production fluid conduit (14);
a second chamber (46); and
a movable divider (38) disposed between and in fluid communication with each of the first chamber (42) and the second chamber (46);
a pressure source (50) configured to be in fluid communication with the second chamber (46); and
the system further comprises a first conduit (74a) configured to be in fluid communication with the first chamber (42) and to convey production fluid expelled from the first chamber (42) to one or more sensors (106a, 106b, 106c) configured to capture data indicative of one or more properties of the production fluid; and
a processor (64) configured to control the pressure source (50) such that, as production fluid is drawn into the first chamber (42), a difference between a force acting on the divider (38) due to pressure within the second chamber (46) ; the system **characterized in that**
a force acting on the divider (38) due to pressure within the first chamber (42) remains within 10% of a force acting on the divider (38) due to pressure within the first chamber (42); and
wherein the processor (64) is configured to control the pressure source (50) such that, as at least on portion of the production fluid is expelled from the first chamber (42) to the one or more sensors (106a, 106b, 106c),
a difference between a force acting on the divider (38) due to pressure within the second chamber (46) remains within 10% of a force acting on the divider (38) due to pressure within the first chamber (42).

7. The system of claim 6, wherein the pressure source (50) comprises a pump (54a) and/or the processor (64) is configured to control the pressure source (50) by controlling a regulator in fluid communication with the pressure source (50), or wherein the divider (38) comprises a piston or a flexible bladder, or the system comprises a heating element (70) configured to heat the container.

8. The system of claim 6, comprising a second conduit (74b) configured to be in fluid communication with the first chamber (42) and to convey production fluid expelled from the first chamber (42) to at least one of the production fluid conduit (14) and a reservoir (78).

9. The system of claim 8, comprising:
one or more valves (82a, 82b) configured to control fluid communication through the first and second conduits (74a, 74b); and
a sensor (86) configured to capture data indicative of a water content of production fluid within the second conduit (74b);
wherein the one or more valves (82a, 82b) are configured to block fluid communication through the second conduit (74b) and allow fluid communication through the first conduit (74a) based, at least in part, on data captured by the sensor (86).

10. The system of claim 6, wherein the one or more properties comprises a TDS content, a pH, an alkalinity, a total hardness, a hydrogen sulfide content, an ammonia content, a hydrocarbon content, a carbon dioxide content, a total metal carbonate content, a total metal sulfate content, a total metals content, a sodium chloride content, a silicate content, an iron content, a calcium content, a sodium content, a magnesium content, a potassium content, a strontium content, a chlorine content, a chloride content, a bicarbonate content, a phosphorous content, a boron content, a barium content, a sulfate content, an iron content, a nickel content, a chromium content, a cobalt content, a molybdenum content, a specific gravity, a conductivity, a saturation index and/or ratio, a resistivity, a pressure, and/or a temperature.

## Patentansprüche

1. Verfahren zur Probenahme und Analyse von Förderfluid aus einem Öl- und Gasbohrloch, wobei das Verfahren Folgendes umfasst:
Aufnehmen von Förderfluid aus der Förderfluidleitung (14) in eine erste Kammer (42) eines Behälters (26), der mit einer Förderfluidleitung (14) gekoppelt ist, wobei der Behälter (26) Folgendes aufweist:
eine zweite Kammer (46); und
einen beweglichen Teiler (38), der zwischen und in Fluidverbindung mit jeder der ersten Kammer (42) und der zweiten Kammer (46) angeordnet ist;
wobei das Aufnehmen von Förderfluid in die erste Kammer (42) das Ziehen von Förderfluid in die erste Kammer (42) mindestens durch Steuern einer Druckquelle (50) in Fluidverbindung mit der zweiten Kammer (46) umfasst, sodass eine Kraft, die aufgrund von Druck in der zweiten Kammer (46) auf den Teiler (38) wirkt, reduziert wird und bleibt, wenn Förderfluid in die erste Kammer (42) gezogen wird,
wobei das Verfahren **dadurch gekennzeichnet ist, dass**
die Kraft, die auf den Teiler wirkt, weniger als aber innerhalb von 10 % einer Kraft ist, die aufgrund von Druck in der ersten Kammer (42) auf den Teiler (38) wirkt; und
wobei das Verfahren ferner Folgendes umfasst:
Ausstoßen mindestens eines Teils des aufgenommenen Förderfluids aus der ersten Kammer (42) mindestens durch Steuern der Druckquelle (50), sodass eine Kraft, die aufgrund von Druck in der zweiten Kammer (46) auf den Teiler (38) wirkt, innerhalb von 10 % einer Kraft bleibt, die aufgrund von Druck in der ersten Kammer (42) auf den Teiler (38) wirkt;
Leiten mindestens eines ersten Teils des ausgestoßenen Förderfluids zu einem oder mehreren Sensoren (106a, 106b, 106c); und
Erfassen von Daten, die eine oder mehrere Eigenschaften mindestens eines Teils des aufgenommenen Förderfluids angeben, mit dem einen oder den mehreren Sensoren (106a, 106b, 106c).

2. Verfahren nach Anspruch 1, umfassend:
Leiten mindestens eines zweiten Teils des ausgestoßenen Förderfluids zu mindestens einem von der Förderfluidleitung (14) und einem Reservoir (78);
wobei das Leiten des ersten und zweiten Teils mindestens teilweise basierend auf Daten durchgeführt wird, die von einem Sensor (86) erfasst werden, der einen Wassergehalt des ersten Teils und/oder des zweiten Teils angibt.

3. Verfahren nach Anspruch 1, umfassend das Kalibrieren mindestens eines des einen oder der mehreren Sensoren (106a, 106b, 106c) mindestens durch Erfassen von Daten, die eine oder mehrere Eigenschaften eines Fluids angeben, mit dem mindestens einen Sensor, wobei mindestens eine der einen oder mehreren Eigenschaften des Fluids bekannt ist.

4. Verfahren nach Anspruch 1, umfassend das Bereitstellen eines oder mehrerer Reagenzien für den Teil des aufgenommenen Förderfluids (14).

5. Verfahren nach Anspruch 4, wobei mindestens eines des einen oder der mehreren Reagenzien auf den pH-Wert und/oder die Alkalität anspricht und Thymolblau, Methylrot, Bromthymolblau, Bromkresolgrün, Bromkresolpurpur und/oder Phenolphthalein umfasst, oder wobei mindestens eines des einen oder der mehreren Reagenzien auf Eisen anspricht und 1,10-Phenanthrolin, 4,7-Diphenyl-1,10-phenanthrolin, 2,4,6-Tris(2-pyridyl)-1,3,5-triazin, 2,2-Bypyridin, Kaliumcyanid und/oder 2,2',2"-Tripyridin umfasst, oder wobei mindestens eines des einen oder der mehreren Reagenzien einen Chelatbildner umfasst.

6. System (10a) zur Probenahme und Analyse von Förderfluid aus einem Öl- und Gasbohrloch, wobei das System (10a) Folgendes umfasst:
einen Behälter (26) mit:
einer ersten Kammer (42), die konfiguriert ist, um in Fluidverbindung mit einer Förderfluidleitung (14) zu stehen;
einer zweiten Kammer (46); und
einem beweglichen Teiler (38), der zwischen und in Fluidverbindung mit jeder der ersten Kammer (42) und der zweiten Kammer (46) angeordnet ist;
eine Druckquelle (50), die konfiguriert ist, um in Fluidverbindung mit der zweiten Kammer (46) zu stehen; und
das System ferner eine erste Leitung (74a) umfasst, die konfiguriert ist, um in Fluidverbindung mit der ersten Kammer (42) zu stehen und Förderfluid, das aus der ersten Kammer (42) ausgestoßen wird, zu einem oder mehreren Sensoren (106a, 106b, 106c) zu befördern, die konfiguriert sind, um Daten zu erfassen, die eine oder mehrere Eigenschaften des Förderfluids angeben; und
einen Prozessor (64), der konfiguriert ist, um die Druckquelle (50) zu steuern, sodass, wenn Förderfluid in die erste Kammer (42) gezogen wird, eine Differenz zwischen einer Kraft, die aufgrund von Druck in der zweiten Kammer (46) auf den Teiler (38) wirkt;
wobei das System **dadurch gekennzeichnet ist, dass**
eine Kraft, die aufgrund von Druck in der ersten Kammer (42) auf den Teiler (38) wirkt, innerhalb von 10 % einer Kraft bleibt, die aufgrund von Druck in der ersten Kammer (42) auf den Teiler (38) wirkt; und
wobei der Prozessor (64) konfiguriert ist, um die Druckquelle (50) zu steuern, sodass, wenn mindestens ein Teil des Förderfluids aus der ersten Kammer (42) zu dem einen oder den mehreren Sensoren (106a, 106b, 106c) ausgestoßen wird, eine Differenz zwischen einer Kraft, die aufgrund von Druck in der zweiten Kammer (46) auf den Teiler (38) wirkt, innerhalb von 10 % einer Kraft bleibt, die aufgrund von Druck in der ersten Kammer (42) auf den Teiler (38) wirkt.

7. System nach Anspruch 6, wobei die Druckquelle (50) eine Pumpe (54a) umfasst und/oder der Prozessor (64) konfiguriert ist, um die Druckquelle (50) durch Steuern eines Reglers in Fluidverbindung mit der Druckquelle (50) zu steuern, oder wobei der Teiler (38) einen Kolben oder eine flexible Blase umfasst oder das System ein Heizelement (70) umfasst, das konfiguriert ist, um den Behälter zu erwärmen.

8. System nach Anspruch 6, umfassend eine zweite Leitung (74b), die konfiguriert ist, um in Fluidverbindung mit der ersten Kammer (42) zu stehen und Förderfluid, das aus der ersten Kammer (42) ausgestoßen wird, zu mindestens einem von der Förderfluidleitung (14) und einem Reservoir (78) zu befördern.

9. System nach Anspruch 8, umfassend:
ein oder mehrere Ventile (82a, 82b), die konfiguriert sind, um die Fluidverbindung durch die erste und die zweite Leitung (74a, 74b) zu steuern; und
einen Sensor (86), der konfiguriert ist, um Daten zu erfassen, die einen Wassergehalt des Förderfluids in der zweiten Leitung (74b) angeben;
wobei das eine oder die mehreren Ventile (82a, 82b) konfiguriert sind, um die Fluidverbindung durch die zweite Leitung (74b) zu blockieren und die Fluidverbindung durch die erste Leitung (74a) mindestens teilweise basierend auf Daten zu ermöglichen, die von dem Sensor (86) erfasst werden.

10. System nach Anspruch 6, wobei die eine oder die mehreren Eigenschaften einen TDS-Gehalt, einen pH-Wert, eine Alkalität, eine Gesamthärte, einen Schwefelwasserstoffgehalt, einen Ammoniakgehalt, einen Kohlenwasserstoffgehalt, einen Kohlendioxidgehalt, einen Gesamtmetallcarbonatgehalt, einen Gesamtmetallsulfatgehalt, einen Gesamtmetallgehalt, einen Natriumchloridgehalt, einen Silicatgehalt, einen Eisengehalt, einen Calciumgehalt, einen Natriumgehalt, einen Magnesiumgehalt, einen Kaliumgehalt, einen Strontiumgehalt, einen Chlorgehalt, einen Chloridgehalt, einen Bicarbonatgehalt, einen Phosphorgehalt, einen Borgehalt, einen Bariumgehalt, einen Sulfatgehalt, einen Eisengehalt, einen Nickelgehalt, einen Chromgehalt, einen Kobaltgehalt, einen Molybdängehalt, ein spezifisches Gewicht, eine Leitfähigkeit, einen Sättigungsindex und/oder ein Sättigungsverhältnis, einen spezifischen Widerstand, einen Druck und/oder eine Temperatur umfassen.

## Revendications

1. Procédé d'échantillonnage et d'analyse d'un fluide de production d'un puits de pétrole et de gaz, le procédé comprenant :
la réception, dans un premier compartiment (42) d'un récipient (26) couplé à une conduite de fluide de production (14), du fluide de production à partir de la conduite de fluide de production (14), le récipient (26) possédant :
un deuxième compartiment (46) ; et
un diviseur mobile (38) disposé entre, et en communication fluidique avec chacun d'entre, le premier compartiment (42) et le deuxième compartiment (46) ;
dans lequel la réception du fluide de production dans le premier compartiment (42) comprend l'aspiration du fluide de production dans le premier compartiment (42), au moins par régulation d'une source de pression (50) en communication fluidique avec le deuxième compartiment (46) de façon qu'une force agissant sur le diviseur (38) et due à la pression à l'intérieur du deuxième compartiment (46) soit réduite ,et reste, pendant que le fluide de production est aspiré dans le premier compartiment (42), le procédé étant **caractérisé en ce que**
la force agissant sur le diviseur est inférieure à, mais à moins de 10 %, d'une force agissant sur le diviseur (38) due à la pression à l'intérieur du premier compartiment (42), et le procédé comprenant en outre :
l'expulsion d'au moins une partie du fluide de production reçu du premier compartiment (42) au moins par régulation de la source de pression (50) de façon qu'une force agissant sur le diviseur (38) due à la pression à l'intérieur du deuxième compartiment (46), reste à moins de 10 % d'une force agissant sur le diviseur (38) due à la pression à l'intérieur du premier compartiment (42) ;
l'envoi vers un ou plusieurs capteurs (106a, 106b, 106c) d'au moins une première partie du fluide de production expulsé ; et
la capture, à l'aide du ou des capteurs (106a, 106b, 106c), de données indicatives d'une ou plusieurs propriétés d'au moins une partie du fluide de production reçu.

2. Procédé selon la revendication 1,
comprenant l'envoi d'au moins une deuxième partie du fluide de production expulsé vers au moins un d'entre la conduite de fluide de production (14) et un réservoir (78) ;
dans lequel l'envoi de la première et de la deuxième partie est réalisé sur la base, au moins en partie, des données capturées par un capteur (86) qui sont indicatives d'une teneur en eau de la première partie et/ou de la deuxième partie.

3. Procédé selon la revendication 1, comprenant l'étalonnage d'au moins le ou les capteurs (106a, 106b, 106c) au moins par capture, à l'aide de l'au moins un capteur, de données indicatives d'une ou plusieurs propriétés d'un fluide, au moins l'une de la ou des propriétés du fluide étant connue.

4. Procédé selon la revendication 1, comprenant l'amenée d'un ou plusieurs réactifs à la partie du fluide de production reçu (14).

5. Procédé selon la revendication 4, dans lequel au moins l'un du ou des réactifs est sensible au pH et/ou à l'alcalinité et comprend du bleu de thymol, du rouge de méthyle, du bleu de bromothymol, du vert de bromocrésol, du pourpre de bromocrésol et/ou de la phénolphtaléine, ou dans lequel au moins l'un du ou des réactifs est sensible au fer et comprend de la 1,10-phénanthroline, de la 4,7-diphényl-1,10-phénanthroline, de la 2,4,6-tris(2-pyridyl)-1,3,5-triazine, de la 2,2-bipyridine, du cyanure de potassium et/ou de la 2,2',2"-tripyridine, ou dans lequel au moins l'un du ou des réactifs comprend un agent chélatant.

6. Système (10a) d'échantillonnage et d'analyse d'un fluide de production d'un puits de pétrole et de gaz, le système (10a) comprenant :
un récipient (26) possédant :
un premier compartiment (42) conçu pour être en communication fluidique avec une conduite de fluide de production (14) ;
un deuxième compartiment (46) ; et
un diviseur mobile (38) disposé entre et en communication fluidique avec chacun du premier compartiment (42) et du deuxième compartiment (46) ;
une source de pression (50) conçue pour être en communication fluidique avec le deuxième compartiment (46) ; et
le système comprenant en outre une première conduite (74a) conçue pour être en communication fluidique avec le premier compartiment (42) et pour envoyer le fluide de production expulsé du premier compartiment (42) vers un ou plusieurs capteurs (106a, 106b, 106c) conçus pour capturer des données indicatives d'une ou plusieurs propriétés du fluide de production ; et
un processeur (64) configuré pour réguler la source de pression (50) de façon que, au fur et à mesure que le fluide de production est aspiré dans le premier compartiment (42), il se crée une différence entre la force agissant sur le diviseur (38), due à la pression à l'intérieur du deuxième compartiment (46) ;
le système étant **caractérisé en ce que**
une force agissant sur le diviseur due à la pression à l'intérieur du premier compartiment (42) est inférieure à, mais à moins de 10 %, d'une force agissant sur le diviseur (38) due à la pression à l'intérieur du deuxième compartiment (46), et le procédé comprenant en outre :
dans lequel le processeur (64) est conçu pour réguler la source de pression (50) de façon que, au fur et à mesure qu'au moins une partie du fluide de production est expulsée du premier compartiment (42) vers le ou les capteurs (106a, 106b, 106c), une différence entre une force agissant sur le diviseur (38), due à la pression à l'intérieur du deuxième compartiment (46), s'écarte de moins de 10 % d'une force agissant sur le diviseur (38), due à la pression à l'intérieur du premier compartiment (42).

7. Système selon la revendication 6, dans lequel la source de pression (50) comprend une pompe (54a) et/ou le processeur (64) est conçu pour réguler la source de pression (50) en régulant un régulateur en communication fluidique avec la source de pression (50), ou dans lequel le diviseur (38) comprend un piston ou une vessie souple, ou le système comprend un élément chauffant (70) conçu pour chauffer le récipient.

8. Système selon la revendication 6, comprenant une deuxième conduite (74b) conçue pour être en communication fluidique avec le premier compartiment (42) et pour envoyer le fluide de production expulsé du premier compartiment (42) vers au moins l'un d'entre la conduite de fluide de production (14) et un réservoir (78).

9. Système selon la revendication 8, comprenant :
une ou plusieurs soupapes (82a, 82b) conçues pour réguler la communication fluidique par les première et deuxième conduites (74a, 74b) ; et
un capteur (86) conçu pour capturer des données indicatives d'une teneur en eau du fluide de production à l'intérieur de la deuxième conduite (74b) ;
dans lequel la ou les soupapes (82a, 82b) sont conçues pour bloquer la communication fluidique par la deuxième conduite (74b) et permettent une communication fluidique par la première conduite (74a) en se fondant au moins en partie sur les données capturées par le capteur (86).

10. Système selon la revendication 6, dans lequel la ou les propriétés comprennent une teneur en TDS, un pH, une alcalinité, une dureté totale, une teneur en sulfure d'hydrogène, une teneur en ammoniac, une teneur en hydrocarbures, une teneur en dioxyde de carbone, une teneur totale en carbonates métalliques, une teneur totale en sulfates métalliques, une teneur totale en métaux, une teneur en chlorure de sodium, une teneur en silicate, une teneur en fer, une teneur en calcium, une teneur en sodium, une teneur en magnésium, une teneur en potassium, une teneur en strontium, une teneur en chlore, une teneur en chlorure, une teneur en bicarbonate, une teneur en composés phosphorés, une teneur en bore, une teneur en baryum, une teneur en sulfate, une teneur en fer, une teneur en nickel, une teneur en chrome, une teneur en cobalt, une teneur en molybdène, une densité, une conductivité, un indice et/ou un taux de saturation, une résistivité, une pression et/ou une température.
